# EUROPEAN PATENT APPLICATION

(11) **EP 1 754 457 A2**
(43) Date of publication of application: **21.02.2007**
(21) Application number: 06445060.4
(22) Date of filing: 16.08.2006
(51) Int. Cl.: A61F 2/38

(54) **Implants and instruments for unicompartmental knee replacement**

(30) Priority: 16.08.2005 US 708385 P
(71) Applicant: Arthrex, Inc., Naples, Florida 34108-1945 (US)
(72) Inventor: Iannarone, Ronald C., Naples, Florida 34119-8703 (US); Young, Dayna Ann, Naples, Florida 34108 (US)
(74) Representative: Janson, Ronny

(57) **Abstract**

An instrument for creating a combination onlay/inlay pocket in an anterior area of a tibia for unicompartmental knee arthroplasty. The pocket includes a cortical curved rim on one side and on the opposing side, an anterior "window" along the straight edge of a tibial spine. A cutting guide holds a cutter, which rotates and plunges back and forth to form the pocket in the tibia. The cutting guide is attached to a double tracks on the surface of a cam track cutting guide. The cutter cuts the tibia dictated by angles formed when the cutting guide rotates by moving along the double tracks of the cam track cutting guide. Once a pocket is formed, a combination onlay/inlay tibial implant is inserted into the pocket.

## Description

### FIELD OF THE INVENTION

The present invention relates to implants and instruments for arthroscopic surgery and, more particularly, to implants and instruments for unicompartmental knee replacement.
BACKGROUND OF THE INVENTION

Partial knee replacement surgery, also called unicompartmental knee arthroplasty, is routinely considered for the treatment of osteoarthritis of the knee joint. Partial knee replacement surgery has generated significant interest because it entails a smaller incision and faster recovery than traditional total joint replacement surgery.

When partial knee replacement is performed, the bone and cartilage on the end of the femur and top of the tibia are removed. This is performed using precise instruments to create exact surfaces to accommodate the implant. A knee replacement implant made of various biocompatible materials such as metal or plastic is then placed to function as a new knee joint. Depending on the condition of the cartilage on the undersurface of the kneecap, this may also be replaced. The knee replacement implant typically comprises (i) a femoral component, made of metal and which fits on the femur, (ii) a tibial component, made of metal and which fits on the tibia, (iii) a patellar component, made of plastic and which replaces the cartilage on the undersurface of the kneecap, and (iv) a plastic insert which fits between the femoral and tibial components.

Currently, there are two general types of unicompartmental knee replacements: those with onlay tibial components and those with inlay tibial components.

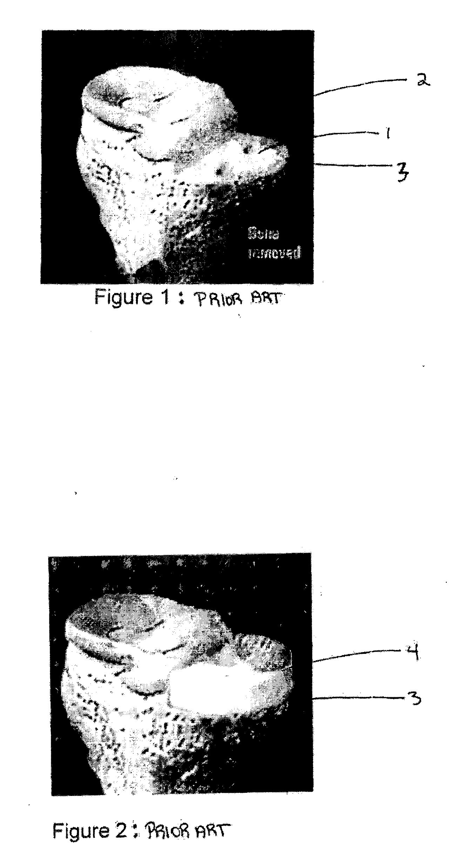

In a unicompartmental knee procedure using an onlay tibial implant, as illustrated in FIG. 1, the tibial plateau 1 is resected by a flat cut. The cut removes the portion of the tibial plateau 1 starting near the tibial spine 2 to the outer rim of the tibia, or cortical rim 3. As shown in FIG. 2, once implanted, the onlay tibial implant 4 rests on the cut on the tibial plateau 1 (FIG.1) and has the benefit of being supported by the cortical rim 3.

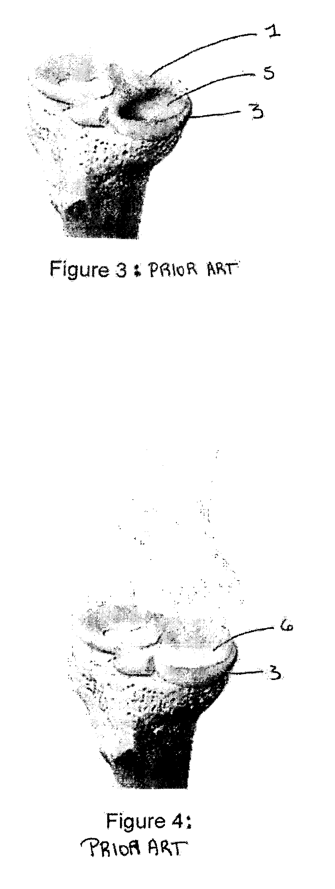

Referring to FIG. 3, in a unicompartmental knee procedure using an inlay tibial implant, the meniscus (not shown) is removed and a pocket of bone is removed from the tibial plateau 1. The removal of the bone creates a pocket 5 in the tibial plateau 1. As illustrated in FIG. 4, an inlay tibial implant 6 rests in the pocket 5 (FIG. 3) and is surrounded by the cortical rim 3.

One drawback of the described onlay and inlay tibial methods is that there is a likelihood that the implants may fail or loosen from the tibia. Accordingly, the need exists for implants and instruments for unicompartmental knee replacement surgery that minimize implant failure or movement.
BRIEF SUMMARY OF THE INVENTION

The present invention overcomes the disadvantages of the prior art and fulfills the needs noted above by providing implants and instruments for resurfacing damaged tibial articulating surfaces in a unicompartmental knee replacement that employs both onlay and inlay tibial component designs.

A combination onlay/inlay pocket is formed in the tibia using an apparatus comprised of a cutter, a cutting guide, and a cam track cutting guide. The cutter forms the pocket by making cuts at angles dictated by the cam track cutting guide. The pocket has a curved outer rim (cortical rim). The inner side of the pocket is a straight edge along the tibial spine and also has an anterior "window." A combination onlay/inlay implant shaped to fit the pocket is inserted into the pocket to complete the tibial component of unicompartmental knee replacement.

These and other features and advantages of the invention will be more apparent from the following detailed description that is provided in connection with the accompanying drawings and illustrated exemplary embodiments of the invention.
BRIEF DESCRIPTION OF THE DRAWINGS

FIG. 1 illustrates a schematic view of a knee undergoing replacement surgery using an onlay tibial implant according to the prior art;

FIG. 2 illustrates another view of the knee of FIG. 1 at a method step subsequent to that of FIG. 1;

FIG. 3 illustrates a schematic view of a knee undergoing replacement surgery using an inlay tibial implant according to the prior art;

FIG. 4 illustrates another view of the knee of FIG. 3 at a method step subsequent to that of FIG. 3;

FIGS. 5a-5b illustrate a universal cutter according to the present invention;

FIG. 6 illustrates an apparatus used to create a combination onlay/inlay tibial pocket according to the present invention;

FIG. 7 illustrates a cutting guide according to the present invention;

FIGS. 8a - 8c illustrate a cam track cutting guide according to the present invention;

FIG. 9 illustrates an alignment rod according to the present invention;

FIG. 10 schematically illustrates the formation of a combination onlay/inlay tibial pocket according to the present invention;

FIG.11 schematically illustrates the formation of a tibial pocket at a stage subsequent to that shown in FIG.10;

FIG. 12 schematically illustrates a schematic view of a tibia with the insertion of the combination onlay/inlay tibial implant according to an embodiment of the present invention;

FIG. 13 illustrates a schematic view of a combination onlay/inlay tibial implant according to an embodiment of the present invention;

FIG. 14 illustrates a schematic view of a tibial trial apparatus according to an embodiment of the present invention.
DETAILED DESCRIPTION OF THE INVENTION

The present invention provides an apparatus for a unicompartmental knee that utilizes the benefits of both the onlay and inlay tibial implants.

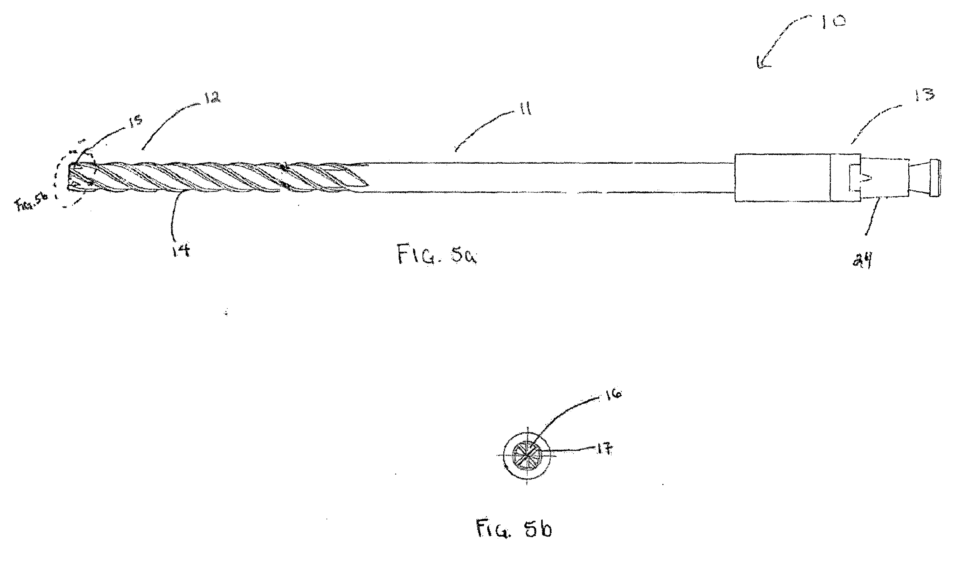

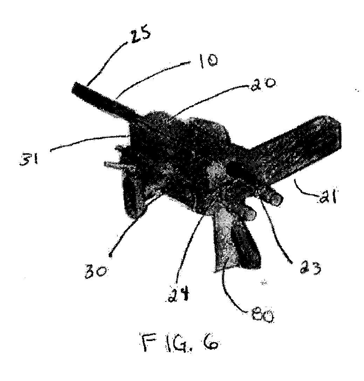

Referring now to the drawings, where like elements are designated by like reference numerals, FIG. 5a - 5b illustrate a universal cutter 10 which is capable of making plunge cuts and side/sweeping cuts in the bone. Referring to FIG. 5a, the cutter 10 features a cylindrical body 11 with a distal end 12 and a proximal end 13. The length of the cutter is about 7 inches. The cutter has a fluted region 14 formed on the distal end 12. This fluted region 14 is about 2.4 inches long. At the tip of the fluted region 14, there are V-shaped openings 15 that are located a certain distance apart. The tip of the fluted region, as shown as a top view in FIG. 5b, includes a region of four grooves 16 divided by stainless steel X-shaped dividers 17.

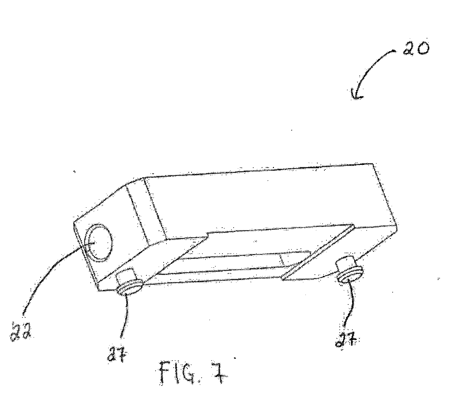

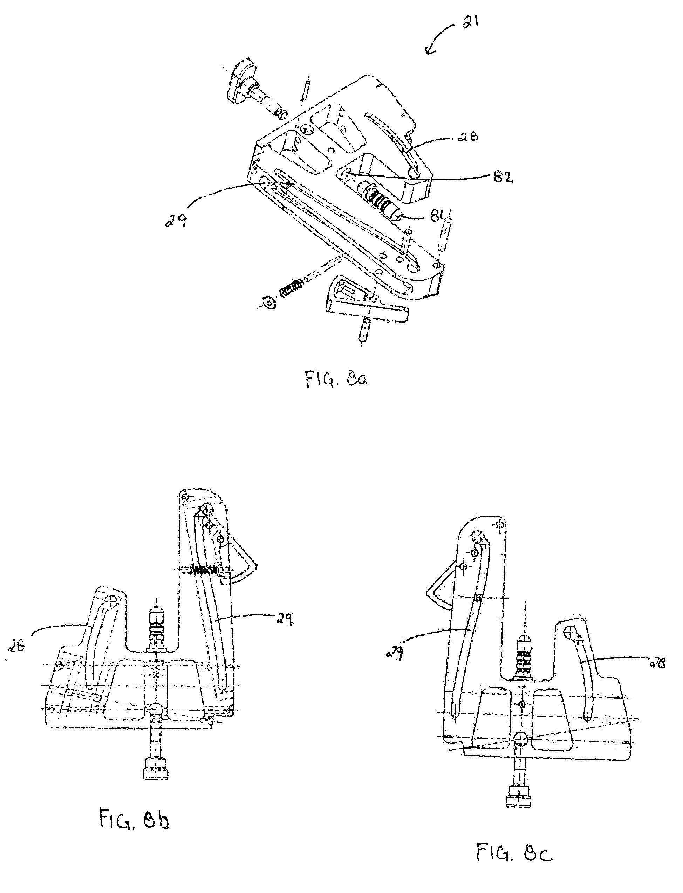

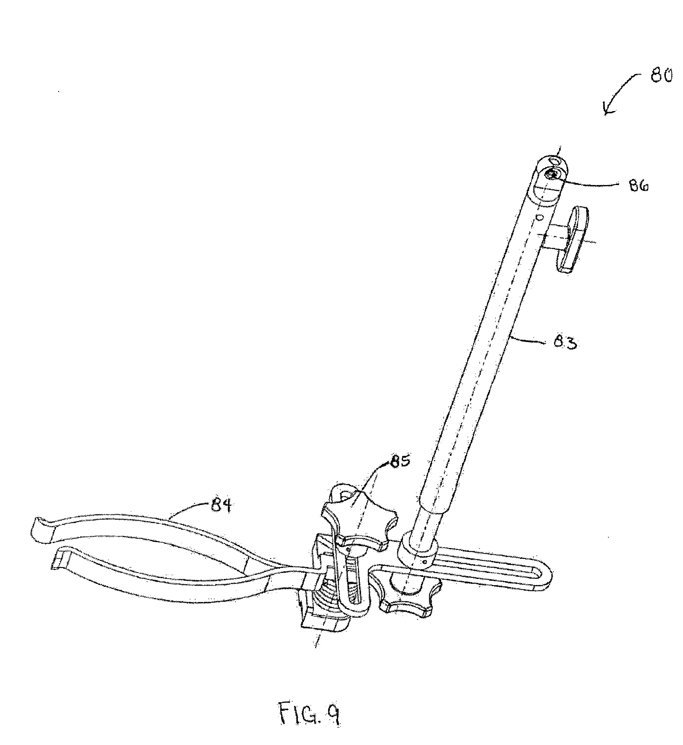

As illustrated in FIG. 6, the cutter 10 is placed in a tibial cutting guide 20. In FIG. 7, an exemplary tibial cutting guide 20 is shown. The cutter 10 is placed in the hole 22 of the cutting guide 20. As illustrated in FIG. 6, the distal end 23 of the cutter comprises a Hudson Quick Connect 24 which secures the cutter 10 within the distal hole 22 (FIG. 7) of the cutting guide 20. The proximal end 25 of the cutter 10, which comprises of the fluted region 14 (FIG. 5a), protrudes from the proximal hole (not shown) of the cutting guide 20. The cutting guide 20 is attached to a cam track cutting guide 21.

Referring to FIG. 8a, a cam track cutting guide 21 is illustrated with various parts unattached. FIGS. 8b - 8c illustrate a top view of the cam track 21 with the parts attached together. The guide pins 27 at the bottom of the cutting guide 20 in FIG. 7 are placed on the tracks 28 and 29 of the cam track 21 illustrated in FIG. 8a. The double tracks (28 and 29) on the cam track 21 aid the cutting guide 20 to move along the tracks to enable the cutter 10 to make plunges in and out of the bone as well as sweeping cuts in the bone. The guide pin 27 (FIG. 7) at the front end 31 of the cutting guide 20 in which the proximal end 25 of the cutter 10 protrudes from (FIG. 6) is placed on the shorter track 28. The other guide pin 27 (FIG. 7) at the back end 30 of the cutting guide 20 in which the distal end 23 of the cutter 10 protrudes from (FIG. 6) is placed on the longer track 29.

The cam track 21 can be rotated depending on which knee is undergoing unicompartmental knee replacement. In FIG. 8b, the cam track 21 is used for the left knee, thus the patient is to the left of the cam track 21. The cam track 21 in FIG. 8c is used for the right knee, thus the patient is to the right of the cam track 21. The longer track 29 is located farther away from the patient and guides the back end 30 of the cutting guide 20 (FIG. 6). The shorter track 28 is placed closer to the patient and guides the front end 31 of the cutting guide 20 (FIG. 6).

Referring to FIG. 6, an alignment rod 80 is attached to the alignment rod attachment pin 81, which is shown in FIG. 8a. The alignment rod attachment pin 81 is shown detached from the cam track cutting guide 21, however, it is attached in the hole 82 on the cam track 21. FIG. 9 illustrates the alignment rod 80. The alignment rod attachment pin 81 is screwed through the hole 86 of the alignment rod 80. The alignment rod 80 comprises of a cylindrical body 83. The bottom portion of the rod 80 includes a clamp 84. The clamp 84 is used to clamp onto a patient's leg or ankle during the knee surgery. The knob 85 is used to change the size of the clamp 84 to accommodate the patient's leg or ankle size. The alignment rod 80 functions by aligning the cam track cutting guide 21 with the proper portion of the tibia that will undergo unicompartmental knee replacement surgery.

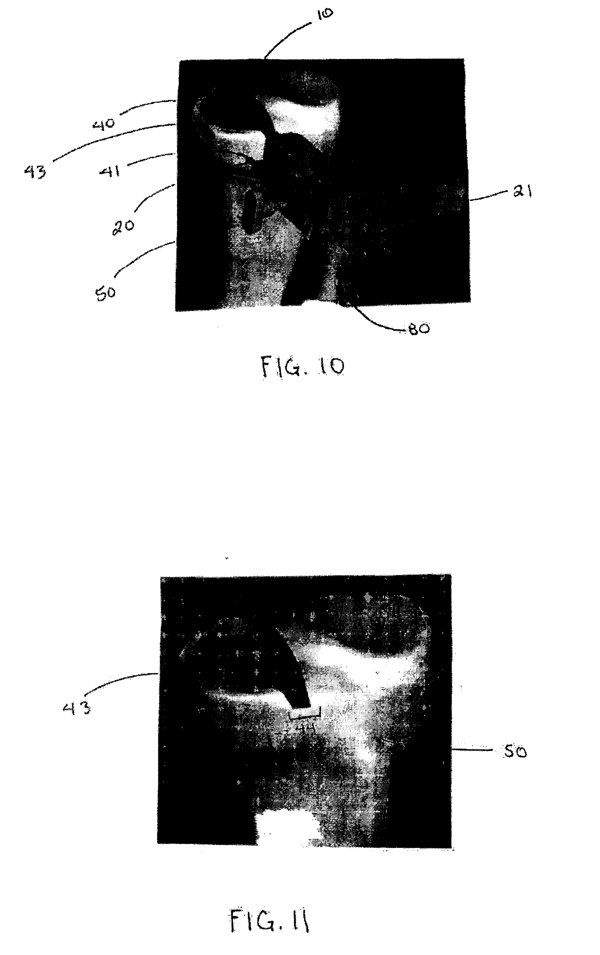

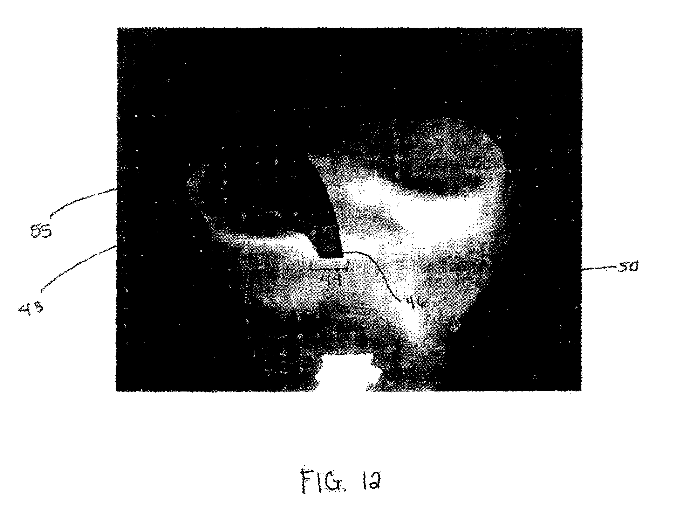

An exemplary method of using the cutter 10, cutting guide 20, and cam track cutting guide 21 to create a combination onlay/inlay tibial pocket 45 of the present invention is described below with reference to FIGS. 10 - 12, which illustrate a schematic view of a knee joint in which unicompartmental knee replacement is performed according to the present invention. In the following embodiment, a combination onlay/inlay tibial pocket 45 with an onlay/inlay tibial implant 55 (shown completed in FIG. 12) is formed in the tibia 50.

The unicompartmental knee tibial implant design of the present invention incorporates the benefits of both the inlay and onlay implants. In the unicompartmental knee procedure of the present invention, as illustrated in FIG. 10, a pocket of bone (not shown) is removed through an anterior area of the tibia 50. A cam track cutting guide 21 is clamped onto the tibia 50 at a position 41 below the tibial plateau 40. A cutter 10 plunges into the anterior tibial plateau 40 at several angles dictated by the cam track guide 21. The cutter 10 also rotates as it cuts into the tibia 50. As the cutting guide 20 moves along the double tracks 28 and 29 (FIG. 8a), the cutting guide 20 rotates into different positions. This enables the cutter 10 to make the plunge cuts into the tibia at different horizontal angles. The initial plunge of the cutter 10 removes most of the pocket of bone. A final sweep with the cutter 10 over the tibial plateau 40 cleans and smoothens the top of the interior curve.

Referring to FIG. 11, the result is a guided cut for a tibial implant 55 (shown in FIG. 12) that leaves a majority of the cortical rim 43 (similar to conventional inlay cuts) while creating an anterior "window" 44 in the tibia 50. The shape of this cut simulates a combination onlay/inlay pocket shape 45 that minimizes failure or movement of a tibial implant 55 (FIG.12). FIG. 12 illustrates a completed method of the onlay/inlay tibial implant 55 placed in the combination onlay/inlay pocket 45 within the tibia 50.

Once implanted, the combination onlay/inlay implant 55 has the benefits of both of the traditional procedures as shown in FIGS. 1-4. The anterior "window" 44 allows the implant 55 the support of resting on cortical bone 46 and along the tibial spine 47 as with an implanted onlay implant. The posterior and medial cortical rim 43 left in tact by the cut allows the implant 55 to be encaptured minimizing implant movement. Further, a femoral component (not shown) may be placed on the femur to complete the unicompartmental knee replacement and allows a painless and smooth glide along the top of the tibial implant 55.

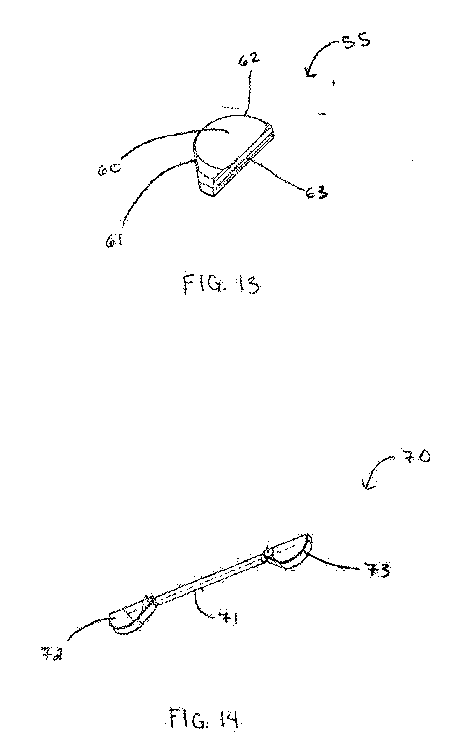

The combination onlay/inlay implant 55 is shown in more detail in FIG. 13. The tibial implant 55 is shaped similar to a top of the tibia. One side of the implant 55 has a curved edge beginning at the distal end 62 that allows the implant 55 to fit along the curve of the cortical rim 43 (FIG. 12). Also, the implant 55 has a slightly slanted proximal end 61 which leads to a smaller width than the distal end 62. Along the opposite side 63 of the implant 55 is a straight edge, which allows the implant 55 to align with the tibial spine 47 (FIG. 12). The top 60 of the tibial implant 55 has a curved indentation, which allows the femoral component (not shown) to glide along the indented top surface 60 of the implant 55.

Before the tibial implant 55 is inserted in the combination onlay/inlay tibial pocket 45, the surgeon may use a tibial trial apparatus 70 as illustrated in FIG. 14. This tibial trial 70 comprises of a cylindrical body 71 with sample tibial implant-shaped heads 72 and 73 on both sides of the body 71. Sample tibial implant 72 is used when the left knee is undergoing surgery and sample tibial implant 73 is used when the right knee is undergoing surgery. The sample tibial implants 72 and 73 are a celcon or acetal copolymer material and are available in various sizes. One side, either sample tibial implant 72 or 73, of the tibial trial 70 is placed in the combination onlay/inlay tibial pocket 45 to determine the size of the implant. The tibial trial 70 may also be used to determine if the tibial pocket 45 has been accurately cut and smoothed.

Once satisfied with the shape and cut of the tibial pocket 45, the surgeon may choose the best size for the tibial implant 55 for insertion into the tibial pocket 45. The tibial implant 55 may be press fit or cemented and/or made of polymer (machined or compression molded). The tibial implant 55 may also be metal backed. For added fixation, the tibial implant 55 may be secured with buttons or screws.

The above description and drawings illustrate preferred embodiments which achieve the objects, features, and advantages of the present invention. It is not intended that the present invention be limited to the illustrated embodiments. Any modification of the present invention which comes within the spirit and scope of the following claims should be considered part of the present invention.

## Claims

1. An implant, comprising:
a curved edge having proximal and distal ends;
a slanted edge at the distal end having a smaller width than the proximal end; and
a straight edge on opposing side of the curved edge and the slanted edge that extends between the proximal and distal ends.

2. The implant according to claim 1, wherein a top surface of the implant has a curved indentation.

3. An apparatus for forming a pocket in bone, comprising:
a cam track cutting guide having double tracks on a surface; a cutting guide attached to the double tracks on the surface of the cam track cutting guide; and
a cutter inserted into the cutting guide.

4. The apparatus according to claim 3, wherein the cutter comprises a cylindrical body having proximal and distal ends.

5. The apparatus according to claim 4, wherein the proximal end comprises a fluted region.

6. The apparatus according to claim 3, wherein the cutter rotates and plunges back and forth in the cutting guide to form the pocket in the bone.

7. The apparatus according to claim 3, wherein the cutting guide rotates to form angles for cutting by moving along the double tracks on the cam track cutting guide.
